(19)

Europäisches
Patentamt
European
Patent Office
Office européen
des brevets

(11) **EP 3 539 991 A1**

(12) **EUROPEAN PATENT APPLICATION**

(43) Date of publication:
**18.09.2019   Bulletin 2019/38**

(21) Application number: **19155042.5**

(22) Date of filing: **06.01.2012**

(51) Int Cl.:
*C07K 16/36* ^(2006.01)      *C12P 21/02* ^(2006.01)
*C12P 21/08* ^(2006.01)      *C12Q 1/02* ^(2006.01)
*G01N 33/53* ^(2006.01)      *A61K 39/395* ^(2006.01)
*A61P 37/06* ^(2006.01)

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority:  **07.01.2011   JP 2011002066**

(62) Document number(s) of the earlier application(s) in
accordance with Art. 76 EPC:
**12731990.3 / 2 662 385**

(71) Applicant: **Chugai Seiyaku Kabushiki Kaisha
Kita-ku
Tokyo 115-8543 (JP)**

(72) Inventors:
• **IGAWA, Tomoyuki
Shizuoka 412-8513 (JP)**

• **NISHISA, Yukiko
Shizuoka 412-8513 (JP)**

(74) Representative: **Lahrtz, Fritz
Patentanwälte
Isenbruck Bösl Hörschler PartG mbB
Prinzregentenstraße 68
81675 München (DE)**

Remarks:
•This application was filed on 01-02-2019 as a
divisional application to the application mentioned
under INID code 62.
•Claims filed after the date of filing of the
application/after the date of receipt of the divisional
application  (Rule 68(4) EPC).

(54) **METHOD FOR IMPROVING PHYSICAL PROPERTIES OF ANTIBODY**

(57)      The present inventors discovered that physicochemical properties of an antibody can be improved by reducing the extracellular matrix-binding activity of the polypeptide.

EP 3 539 991 A1

**Description**

Background Art

When a polypeptide is administered as a drug, its *in vivo* kinetics such as blood plasma kinetics undergoes various changes depending on the physicochemical properties of the polypeptide. Examples of physicochemical factors that determine the *in vivo* kinetics of a polypeptide include degradability in blood plasma (degradation of the peptide chain region, and degradation of the sugar chain region), solubility, antigenicity, metabolism in the liver, and affinity for plasma proteins (albumin and such), but are not limited thereto.

When polypeptides are used as drugs, even if molecules that demonstrate high activity *in vitro* are obtained through large-scale screening, they cannot be expected to be highly potent when administered *in vivo* if their *in vivo* kinetics is poor. Therefore, improving their *in vivo* kinetics is an important task in drug development.

Generally, when verifying the *in vivo* kinetics of such polypeptides, one tries methods of administering the polypeptides to various small animals (mice and rats). However, such time-consuming methods are not suitable for drug development which requires rapidity. Furthermore, the technique is also not suitable for carrying out large-scale screening which is required for drug search, and the labor intensiveness forces the scale to be small.

Therefore, if there are *in vitro* methods that can be efficiently carried out at low costs to predict the *in vivo* kinetics of polypeptides with high reliability, they will be very useful in drug development.

Investigation of multiple antibody molecules has revealed that the speed of their degradation by proteases *in vitro* correlates with their blood half-life *in vivo* (Patent Document 1). There are many factors besides protease resistance that determine the *in vivo* kinetics of polypeptides; and since the effects of various factors on the *in vivo* kinetics of polypeptides are different depending on the characteristics and type of the polypeptides, it is important to examine in detail the correlation between *in vivo* kinetics and multiple different *in vitro* parameters.

The blood concentration of a subcutaneously administered VEGF-Trap molecule, which is a fusion protein of the VEGF receptor and Fc, was examined (Non-Patent Document 1). Since the VEGF-Trap molecule which has a high isoelectric point has low concentration in blood plasma when administered subcutaneously, it has low bioavailability. A VEGF-Trap molecule modified by reducing its isoelectric point with amino acid modifications shows high concentration in blood plasma, and thus its bioavailability can be improved. Furthermore, since the change in bioavailability correlates with the strength of extracellular matrix binding, when a VEGF-Trap molecule is administered subcutaneously, the bioavailability of the molecule is found to depend on the strength of extracellular matrix binding in the subcutaneous region.

As such, while the bioavailability of polypeptides has been reported to correlate with the strength of binding of the polypeptides to extracellular matrix, it is unclear as to how plasma half-life and various other parameters (for example, plasma clearance, immunogenicity, and solubility), which are important in making polypeptides into pharmaceutical agents, correlate with the strength of binding of the polypeptides to extracellular matrix. Furthermore, conventional methods for evaluating the strength of extracellular matrix binding using ELISA lack sensitivity.

Prior art documents of the present invention are indicated below.

[Prior Art Documents]

[Patent Document]

[Patent Document 1] WO 2009/128931

[Non-patent Document]

[Non-patent Document 1] Proc. Natl. Acad. Sci., 2002, 99(17), 11393-11398.

Summary of the Invention

[Problems to be Solved by the Invention]

The present invention was achieved in view of the above circumstances. An objective of the present invention is to provide methods for improving physicochemical properties of polypeptides, in particular, methods for improving plasma kinetics, methods for reducing immunogenicity, and methods for improving solubility of polypeptides.

[Means for Solving the Problems]

The present inventors performed dedicated research on methods for improving physicochemical properties of polypeptides. As a result, the present inventors discovered that the physicochemical properties of polypeptides, in particular, plasma kinetics, immunogenicity, and solubility can be improved by modifying at least one amino acid residue of the polypeptides, such that their extracellular matrix-binding activity is lowered.

Accordingly, the present invention relates to methods for improving physicochemical properties of polypeptides, in particular, methods for improving plasma kinetics or methods for reducing immunogenicity when administering the polypeptides as drugs, or methods for improving solubility of the polypeptides when they are made into solution formulations, methods for producing these polypeptides, and methods for evaluating polypeptides produced by these manufacturing methods as well as physicochemical properties of the polypeptides. Specifically, the present invention relates to the following:

[1] a method for improving a physicochemical property of a polypeptide by reducing its extracellular matrix-binding activity;

[2] the method of [1], wherein the improvement of a physicochemical property is at least one selected from improvement of plasma kinetics, reduction of immunogenicity, and improvement of solubility;

[3] the method of [1] or [2], wherein reduction of the extracellular matrix-binding activity is accomplished by modifying at least one amino acid residue of the polypeptide;

[4] the method of any one of [1] to [3], wherein the extracellular matrix-binding activity is measured by an electrochemiluminescence method;

[5] the method of any one of [1] to [4], wherein the polypeptide is a polypeptide having antigen-binding activity;

[6] the method of [5], wherein the polypeptide having antigen-binding activity is an antibody;

[7] a method for producing a polypeptide with improved physicochemical property, which comprises the step of modifying at least one amino acid residue of a polypeptide such that its extracellular matrix-binding activity is reduced;

[8] a method for producing a polypeptide with improved physicochemical property, which comprises the steps of:

(a) modifying at least one amino acid residue of a polypeptide;
(b) measuring the extracellular matrix-binding activity of the polypeptide modified in step (a); and
(c) selecting a polypeptide whose extracellular matrix-binding activity is reduced in comparison to before the modification;

[9] the production method of [8], which further comprises the steps of:

(d) obtaining a gene encoding the polypeptide selected in step (c) of [8]; and
(e) producing a polypeptide using the gene obtained in step (d);

[10] the production method of [9], which further comprises the step of:
(f) modifying at least one amino acid residue of the polypeptide obtained in step (e) of [9], and repeatedly performing steps (b) to (e) mentioned above;

[11] a method for producing a polypeptide having a superior physicochemical property to that of a standard polypeptide by using a polypeptide having a predetermined physicochemical property as the standard polypeptide, wherein the method comprises the steps of:

(a) contacting the standard polypeptide and a test polypeptide or a test polypeptide library with an extracellular matrix;
(b) measuring the extracellular matrix-binding activity of the polypeptides contacted in step (a);
(c) selecting from the polypeptides measured in step (b), a polypeptide whose extracellular matrix-binding activity is lower than that of the standard polypeptide;
(d) obtaining a gene encoding the polypeptide selected in step (c); and
(e) producing a polypeptide using the gene obtained in step (d);

[12] the production methods of [7] to [11], wherein the improvement of a physicochemical property is at least one selected from improvement of plasma kinetics, reduction of immunogenicity, and improvement of solubility;

[13] the production method of any one of [7] to [12], wherein the extracellular matrix-binding activity is measured by an electrochemiluminescence method;

[14] the production method of any one of [7] to [13], wherein the polypeptide is a polypeptide having antigen-binding activity;

[15] the production method of [14], wherein the polypeptide having antigen-binding activity is an antibody;

[16] a polypeptide produced by the production method of any one of [7] to [15];

[17] a method of screening for a polypeptide having a superior physicochemical property to that of a standard polypeptide by using a polypeptide having a predetermined physicochemical property as the standard polypeptide, wherein the method comprises the steps of:

> (a) contacting the standard polypeptide and a test polypeptide or a test polypeptide library with an extracellular matrix;
> (b) measuring the extracellular matrix-binding activity of the polypeptides contacted in step (a); and
> (c) selecting from the polypeptides measured in step (b), a polypeptide whose extracellular matrix-binding activity is lower than that of the standard polypeptide;

[18] the screening method of [17], wherein the improvement of a physicochemical property is at least one selected from improvement of plasma kinetics, reduction of immunogenicity, and improvement of solubility;
[19] the screening method of [17] or [18], wherein the extracellular matrix-binding activity is measured by an electrochemiluminescence method;
[20] the screening method of any one of [17] to [19], wherein the polypeptide is a polypeptide having antigen-binding activity;
[21] the screening method of [20], wherein the polypeptide having antigen-binding activity is an antibody;
[22] a method for evaluating a physicochemical property of a test polypeptide by measuring the extracellular matrix-binding of the test polypeptide and comparing it to the extracellular matrix-binding activity of another polypeptide whose physicochemical property is predetermined;
[23] the method of [22], wherein the physicochemical property is at least one selected from plasma kinetics, immunogenicity, and solubility of the polypeptide; and
[24] the method of [22] or [23], wherein measurement of the extracellular matrix-binding activity is measurement by an electrochemiluminescence method.

[Effects of the Invention]

The present invention provides methods that reduce the extracellular matrix-binding of polypeptides having a desired function for improving physicochemical properties of the polypeptides or for producing polypeptides with improved physicochemical properties. In particular, improvement of the physicochemical properties of polypeptides may be improvement of plasma kinetics, reduction of immunogenicity, or improvement of solubility; and therefore polypeptides suitable for use as pharmaceuticals can be provided. Moreover, the physicochemical properties of a test polypeptide can be evaluated by comparing the extracellular matrix-binding activity of a polypeptide having predetermined physicochemical properties with that of the test polypeptide.

Brief Description of the Drawings

Fig. 1 shows the relationship between the extracellular matrix binding of antibodies (MabA1 to A3) and their half-life (d) when the antibodies are administered to normal mice.
Fig. 2 shows ELISA measurements of the extracellular matrix binding of antibodies (MabB1, MabB2, and MabB3).
Fig. 3 is a graph showing the results of evaluating the plasma kinetics of antibodies (MabB1 to B3) in normal mice by the method demonstrated in Example 1 (intravenous administration).
Fig. 4 is a graph showing the results of evaluating the plasma kinetics of antibodies (MabB1 to B3) in normal mice by the method demonstrated in Example 1 (subcutaneous administration).
Fig. 5 shows ECL measurements of the extracellular matrix binding of antibodies (MabB2 and Mab B3).
Fig. 6 shows the relationship between the extracellular matrix binding of antibodies (MabC7 to MabC9) and their half-life (d) when the antibodies are administered to human FcRn transgenic mice.
Fig. 7 is a graph demonstrating the extracellular matrix binding of antibody polypeptides (MabC1 to MabC6) and T-cell assay response rate.
Fig. 8 is a graph showing the relationship between the number of days of administration and the emergence of anti-antibody upon administration of antibodies (MabB2 and MabB3).
Fig. 9 is a graph showing the relationship between the extracellular matrix binding of antibodies (MabD1 to MabD30) and their solubility.
Fig. 10 depicts a graph showing the relationship between the extracellular matrix binding of antibodies (MabD31 and MabD32) and their solubility.

Mode for Carrying Out the Invention

The present invention provides methods for improving the physicochemical properties of polypeptides. More specifically, the present invention provides methods for improving physicochemical properties of polypeptides by reducing the extracellular matrix-binding activity of the polypeptides. In particular, the present invention provides methods for improving plasma kinetics and methods for reducing immunogenicity when polypeptides are administered *in vivo*; and methods for improving solubility when polypeptides are made into solution formulations.

Furthermore, the present invention provides methods for evaluating the physicochemical properties of a test polypeptide by comparing the extracellular matrix-binding activity of the test polypeptide to the extracellular matrix-binding activity of a polypeptide with predetermined physicochemical properties.

The "polypeptides" of interest in the present invention are not particularly limited, and they are preferably polypeptides that have a specific function when administered *in vivo*; and in particular, polypeptides that are effective as pharmaceuticals are preferred. Examples of such polypeptides include antibodies, hormones, and cytokines.

In the present invention, examples of polypeptides having antigen-binding activity include antibodies. Preferred examples of antibodies of the present invention include IgG antibodies. When an IgG antibody is used as the antibody, its type is not limited; and IgGs of isotypes (subclasses) such as IgG1, IgG2, IgG3, and IgG4 may be used. Furthermore, antibody constant regions may be included in the polypeptides of the present invention, and amino acid modifications may be introduced into the constant-region portions.

When the subject "polypeptide" of the present invention is an antibody, it may be an antibody derived from any animal, for example, a mouse antibody, human antibody, rat antibody, rabbit antibody, goat antibody, or camel antibody. Furthermore, it may be a modified antibody with substitutions in the amino acid sequence, for example, a chimeric antibody, in particular, a humanized antibody. The antibody may also be bispecific antibodies, antibody modification products linked with various molecules, polypeptides containing antibody fragments, and the like.

"Chimeric antibodies" are antibodies generated by combining sequences derived from different animals. Specifically, the chimeric antibody includes, for example, antibodies having the heavy and light chain variable (V) regions of a mouse antibody and the heavy and light chain constant (C) regions of a human antibody.

"Humanized antibodies", also referred to as reshaped human antibodies, are antibodies in which the complementarity determining regions (CDRs) of an antibody derived from a mammal other than human, for example, a mouse antibody, are transplanted into the CDRs of a human antibody. Methods for identifying CDRs are known (Kabat et al., Sequence of Proteins of Immunological Interest (1987), National Institute of Health, Bethesda, Md.; Chothia et al., Nature (1989) 342:877). Their typical genetic recombination technologies are also known (see European Patent Application EP 125023; and WO 96/02576).

When the polypeptides of the present invention are chimeric antibodies or humanized antibodies, the C regions of these antibodies are preferably derived from human antibodies. For example, $C\gamma1$, $C\gamma2$, $C\gamma3$, and $C\gamma4$ can be used as the H-chain, while $C\kappa$ and $C\lambda$ can be used for the L-chain. Meanwhile, amino acid modifications can be introduced into the human antibody C region as required to increase or decrease $Fc\gamma$ receptor binding, or to improve antibody production or stability. A chimeric antibody of the present invention preferably comprises a variable region of an antibody derived from a mammal other than human and a constant region derived from a human antibody. On the other hand, a humanized antibody preferably comprises CDRs of an antibody derived from a mammal other than human, and FRs and C regions derived from a human antibody. The constant regions derived from a human antibody preferably contain the human FcRn-binding region, and examples of such antibodies include IgG (IgG1, IgG2, IgG3, and IgG4). The constant regions used in the humanized antibodies of the present invention may be constant regions for antibodies of any isotype. A constant region derived from human IgG1 is preferably used; however, it is not limited thereto. The human antibody-derived FRs used in the humanized antibodies are not particularly limited either, and may be derived from an antibody of any isotype.

The variable and constant regions of the chimeric and humanized antibodies of the present invention may be modified by deletion, substitution, insertion, and/or addition, as long as they exhibit the same binding specificity as that of the original antibodies.

A bispecific antibody refers to an antibody carrying variable regions that recognize different epitopes within the same antibody molecule. The bispecific antibody may be an antibody that recognizes two or more different antigens or an antibody that recognizes two or more different epitopes on the same antigen.

Furthermore, polypeptides containing antibody fragments include, for example, Fab fragments, F(ab')2 fragments, scFv (Nat Biotechnol. 2005 September; 23(9):1126-36) domain antibodies (dAb) (WO 2004/058821, WO 2003/002609), scFv-Fc (WO 2005/037989), dAb-Fc, and Fc fusion polypeptides. For molecules containing an Fc region, the Fc region can be used as a human FcRn-binding domain. Alternatively, a human FcRn-binding domain may be fused to these molecules.

Further, the polypeptides in the present invention may be antibody-like molecules. An antibody-like molecule (scaffold molecule or peptide molecule) is a molecule that can exhibit functions by binding to a target molecule (Current Opinion in Biotechnology 2006, 17:653-658; Current Opinion in Biotechnology 2007, 18:1-10; Current Opinion in Structural Biology 1997, 7:463-469; Protein Science 2006, 15:14-27), and includes, for example, DARPins (WO 2002/020565), Affibody (WO 1995/001937), Avimer (WO 2004/044011; WO 2005/040229), and Adnectin (WO 2002/032925). Even for these antibody-like molecules, it is possible to improve physicochemical properties of the antibody molecules by reducing their extracellular matrix-binding activities.

In the present invention, "physicochemical properties" of polypeptides refers to plasma kinetics or immunogenicity when the polypeptides are administered *in vivo*, and solubility when the polypeptides are made into solution formulations. In the present invention, "improvement of physicochemical properties" only requires improvement of any one of these physicochemical properties; and in particular, improvement of any one of plasma kinetics, immunogenicity, and solubility which are important when using the polypeptides as pharmaceuticals is preferred.

Furthermore, the method for measuring "extracellular matrix-binding" is not particularly limited, and measurements can be carried out using an ELISA system that detects binding between a polypeptide and extracellular matrix by adding the polypeptide to an extracellular matrix-immobilized plate, and adding a labeled antibody against the polypeptide. In particular, a measurement method using the electrochemiluminescence (ECL) method is preferred since it enables detection of extracellular matrix-binding ability with higher sensitivity. Specifically, a mixture of polypeptide and ruthenium antibody is added to an extracellular matrix-immobilized plate, and the binding between the polypeptide and extracellular matrix can be measured using an ECL system that measures the electrochemiluminescence of ruthenium. The concentration of polypeptide added can be arbitrarily set, and it is preferable to add high concentrations to increase the detection sensitivity of extracellular matrix-binding. While the extracellular matrix used in the present invention may be plant-derived or animal-derived as long as it contains glycoproteins such as collagen, proteoglycans, fibronectin, laminin, entactin, fibrin, and perlecan, animal-derived extracellular matrix is preferred in the present invention; and for example, extracellular matrix derived from animals such as humans, mice, rats, monkeys, rabbits, and dogs can be used. In particular, for monitoring the improvement of plasma kinetics in humans, naturally-occurring human extracellular matrices derived from humans are preferred. Furthermore, the condition for evaluating the binding of polypeptides to extracellular matrix is desirably in a neutral range near pH 7.4 (physiological condition), but it does not necessarily have to be in the neutral range, and evaluation may also be performed in the acidic range (near pH 6.0). Furthermore, when evaluating the binding of a polypeptide to extracellular matrix, antigen molecules bound by the polypeptide can be made to coexist for evaluation of the binding of the polypeptide/antigen molecule complex to the extracellular matrix.

In the present invention, "improvement of plasma kinetics" can be rephrased as "improvement of plasma pharmacokinetics", "improvement of plasma retention", "excellent plasma retention", or "extending plasma retention", and these phrases are used in the same meaning.

In the present invention, "improvement of plasma kinetics" means extension of the time from administration of a polypeptide of the present invention to animals such as humans, mice, rats, monkeys, rabbits, and dogs to disappearance from the plasma (for example, until the polypeptide of the present invention reaches a state where it can no longer return to the plasma because it is degraded in cells and such). That is, "improvement of plasma kinetics" can be confirmed by measuring any one parameter such as increase of half-life in blood plasma, increase in the average retention time in blood plasma, or decrease in plasma clearance (Pharmacokinetics: Enshu niyoru Rikai (Understanding through Exercises), (Nanzando)). For example, when a test polypeptide is administered to mice, rats, monkeys, rabbits, dogs, humans, and such, the plasma concentration of the polypeptide is measured, and each parameter is calculated. For example, when the half-life in plasma is prolonged or when the average retention time in plasma is prolonged, the plasma kinetics of the polypeptide can be said to have improved. These parameters can be measured using methods known to those skilled in the art, and for example, they can be assessed appropriately by noncompartmental analysis using the pharmacokinetics analysis software WinNonlin (Pharsight) according to the attached instruction manual.

In the present invention, improvement of plasma kinetics in humans is preferred. When it is difficult to measure plasma kinetics in humans, plasma kinetics in humans can be predicted based on plasma kinetics in mice (for example, normal mice, human antigen-expressing transgenic mice, human FcRn-expressing transgenic mice) or monkeys (for example, cynomolgus monkeys).

In the present invention, "reduction of immunogenicity" means suppression of inflammatory cytokine production or suppression of cell growth when a polypeptide of the present invention is added to immune cells *in vitro*, or reduction of intensity or frequency at which antibodies appear against the polypeptide when the polypeptide is administered *in vivo*. Specifically, when a polypeptide is added to PBMCs *in vitro,* cell growth can be measured on a spectrophotometer by utilizing the incorporation of bromodeoxyuridine (BrdU), a substrate that develops a blue color, into newly synthesized DNA in actively proliferating cells (Baker MP, Carr FJ.Curr Drug Saf. 2010, 5(4):308-13). Furthermore, antibodies that appear *in vivo* when a polypeptide is administered *in vivo* can be detected and measured by methods known to those skilled in the art, such as Sandwich-ELISA using the polypeptide or ELISA using polypeptide-immobilized plates. Cytokine production can be measured by methods using Sandwich ELISA which uses antibodies directed to cytokines, and such. More specifically, immunogenicity can be measured according to a method similar to that of Example 4.

In the present invention, "improvement of solubility" means increase of solubility in a buffer in the presence of polyethylene glycol (PEG) (Guiotto, et al. (2004) Bioorg. Med. Chem. 12, 5031-5037) and increase of solubility actually measured in a buffer. It is possible to select any type of buffer for measuring solubility. Specifically, for example, solubility can be measured by following a method similar to that of Example 6.

Amino acid residue modifications that will reduce the binding of a polypeptide of the present invention to the extracellular matrix are not particularly limited; however, in the present invention, modification of amino acids located on the surface of polypeptides having antigen-binding activity is preferred. For example, extracellular matrix-binding is reduced by modifying at least one amino acid residue of the polypeptide. In this case, it is preferable to consider minimizing the effects of the modification on other activities of the polypeptide.

Therefore, in the present invention, for example, when the polypeptide of the present invention is an antibody, it is preferable to modify the variable-region amino acids exposed on the antibody surface since residues exposed on the surface of an antibody variable-region portion are mainly involved in extracellular matrix binding. Variable-region amino acids exposed on the antibody surface can be found by methods known to those skilled in the art using antibody homology models.

Modification of amino acid residues can be carried out as described below by modifying one or multiple nucleotides in the polypeptide-encoding DNA and expressing the DNA in host cells. Those skilled in the art can easily determine the number, position, and type of nucleotides to be modified according to the type of post-modification amino acid residues.

Modification in the present invention means any one of or a combination of substitutions, deletions, additions, and insertions.

In the present invention, "modification of nucleotides" means performing mutation treatment or gene manipulation to insert, delete, or substitute at least one nucleotide in a DNA such that polypeptides encoded by the DNA will have the amino acid residues of interest. More specifically, this includes substitution of codons encoding the original amino acid residues with codons that encode the desired amino acid residues. Such nucleotide modifications can be carried out using methods such as site-directed mutagenesis (see for example, Kunkel (1985) Proc. Natl. Acad. Sci. USA 82: 488), PCR mutagenesis, and cassette mutagenesis. When the polypeptide of the present invention is an antibody, in general, the antibody variant with improved biological properties has an amino acid sequence homology and/or similarity of 70% or higher, more preferably 80% or higher, and even more preferably 90% or higher (for example, 91%, 92%, 93%, 94%, 95%, 96%, 97%, 98%, 99%, or higher) to the amino acid sequence of the variable region of the original antibody. Herein, sequence homology and/or similarity is defined as the ratio of amino acid residues that are homologous (same residue) or similar (amino acid residues classified into the same group based on the general properties of amino acid side chains) to the original antibody residues after necessary sequence alignment and gap introduction to maximize the value of sequence homology. Generally, naturally-occurring amino acid residues are classified into the following groups based on the characteristics of their side chains: (1) hydrophobic: alanine, isoleucine, valine, methionine, and leucine; (2) neutral hydrophilic: asparagine, glutamine, cysteine, threonine, and serine; (3) acidic: aspartic acid, and glutamic acid; (4) basic: arginine, histidine, and lysine; (5) residues that affect the orientation of the chain: glycine and proline; and (6) aromatic: tyrosine, tryptophan, and phenylalanine.

DNAs that encode polypeptides modified as described are cloned (inserted) into suitable vectors and then introduced into host cells. These vectors are not particularly limited as long as the inserted nucleic acids such as DNAs are stably maintained. For example, when *Escherichia coli* is used as a host, examples include cloning vectors. The cloning vectors are preferably pBluescript vectors (manufactured by Stratagene) and such; however, various commercially available vectors may be used.

When vectors are used for the purpose of producing polypeptides of the present invention, expression vectors are particularly useful. There is no particular limitation on the expression vectors, as long as they can express polypeptides in test tubes, *E. coli,* cultured cells, or individual organisms. For example, preferred vectors include pBEST vectors (manufactured by Promega) for expression in test tubes, pET vectors (manufactured by Invitrogen) for expression in *E. coli,* the pME18S-FL3 vector (GenBank Accession No. AB009864) for expression in cultured cells, and the pME18S vector (Mol Cell Biol. 8:466-472 (1988)) for expression in individual organisms. Insertion of DNAs into vectors can be performed by standard methods such as ligase reactions using restriction enzyme sites (Current protocols in Molecular Biology edit. Ausubel et al. (1987) Publish. John Wiley & Sons. Section 11.4-11.11).

There is no particular limitation on the above-mentioned host cells, and various host cells are used depending on the purpose. Examples of cells used for expressing polypeptides include bacterial cells (for example, *Streptococcus, Staphylococcus, E. coli, Streptomyces,* and *Bacillus subtilis*), fungal cells (for example, yeast and *Aspergillus*), insect cells (for example, *Drosophila* S2 and *Spodoptera* SF9), animal cells (for example, CHO, COS, HeLa, C127, 3T3, BHK, HEK293, Bowes melanoma cell), and plant cells. Vectors can be introduced into host cells using known methods such as the calcium phosphate precipitation method, electroporation method (Current protocols in Molecular Biology edit. Ausubel et al. (1987) Publish. John Wiley & Sons. Section 9.1-9.9), lipofection method, and microinjection method.

To secrete host cell-expressed polypeptides into the lumen of the endoplasmic reticulum, periplasmic space or extracellular environment, suitable secretion signals can be incorporated into the polypeptides of interest. These signals may be endogenous to the polypeptides of interest or may be heterologous signals.

When polypeptides are secreted into a culture medium, the medium is collected to recover the expression products. When the polypeptides are produced inside cells, the cells are first lysed and then the polypeptides are collected.

The polypeptides can be collected and purified from recombinant cell cultures using known methods, including ammonium sulfate or ethanol precipitation, acidic extraction, anion or cation exchange chromatography, phosphocellulose chromatography, hydrophobic interaction chromatography, affinity chromatography, hydroxyapatite chromatography, and lectin chromatography.

In the present invention, polypeptides with improved physicochemical properties can be obtained by deciding on a polypeptide that will serve as a standard for the physicochemical properties, and selecting polypeptides having an extracellular matrix-binding ability lower than that of this polypeptide from pre-existing antibodies, pre-existing libraries (phage libraries and such), antibodies or libraries produced from hybridomas obtained from immunization of animals or B cells from immunized animals, antibodies or libraries produced by randomly modifying the amino acids of these antibodies or libraries, or by modifying the amino acids of the polypeptide serving as the standard to lower the extracellular matrix-binding ability. Amino acid modifications can be carried out according to the above description. Methods for evaluating improvement of physicochemical properties can be carried out by following the methods described in the Examples.

Furthermore, the present invention provides methods for producing polypeptides with improved physicochemical properties. More specifically, the present invention provides methods for producing polypeptides with reduced extracellular matrix-binding activity. Furthermore, the present invention provides methods for producing polypeptides with improved plasma kinetics, reduced immunogenicity, or improved solubility by reducing the extracellular matrix-binding activity. Furthermore, the present invention provides methods for producing polypeptides having antigen-binding activity that are specifically useful for use as pharmaceutical compositions, in particular, antibodies.

Specifically, the present invention provides a method for producing polypeptides comprising the following steps of:

(a) modifying at least one amino acid residue of a polypeptide;
(b) measuring extracellular matrix-binding activity of the polypeptide modified in step (a) mentioned above; and
(c) selecting a polypeptide whose extracellular matrix-binding activity is reduced as compared to before the modification.

Steps (a) and (b) may be repeated two times or more. There are no particular limitations on the number of times steps (a) and (b) are repeated, but it is usually ten times or less.

The above-mentioned production method may further include the following steps of:

(d) obtaining a gene encoding the polypeptide selected in step (c) mentioned above; and
(e) producing a polypeptide using the gene obtained in step (d) mentioned above.

The above-mentioned production method may further include the following step of:
(f) modifying at least one amino acid residue of the polypeptide obtained in step (e) mentioned above, and repeatedly performing steps (b) to (e) mentioned above.

The number of times steps (b) to (e) are repeated in step (f) is not particularly limited, but it is usually ten times or less.

Furthermore, the present invention provides methods for producing polypeptides with superior physicochemical properties to those of a polypeptide with predetermined physicochemical properties by using the polypeptide as a standard. More specifically, the present invention provides methods for producing polypeptides with improved plasma kinetics, reduced immunogenicity, or improved solubility in comparison to a polypeptide with predetermined plasma kinetics, immunogenicity, or solubility, by using the extracellular matrix-binding activity of the polypeptides as an indicator. Furthermore, the present invention provides methods for producing polypeptides having antigen-binding activity that are specifically useful for use as pharmaceutical compositions, in particular, antibodies.

Specifically, the present invention provides a method for producing polypeptides with superior physicochemical properties to those of a standard polypeptide by using a polypeptide with preliminary known physicochemical properties as a standard polypeptide, wherein the method comprises the steps of:

(a) contacting the standard polypeptide and a test polypeptide or a test polypeptide library with an extracellular matrix;
(b) measuring the extracellular matrix-binding activities of the polypeptides contacted in step (a);
(c) selecting from the polypeptides measured in step (b), a polypeptide with lower extracellular matrix-binding activity than that of the standard polypeptide;
(d) obtaining a gene encoding the polypeptide selected in step (c); and
(e) producing a polypeptide using the gene obtained in step (d).

Steps (a) to (c) may be repeated two times or more. The number of times steps (a) to (c) are repeated is not particularly limited, but it is usually ten times or less.

Furthermore, the present invention provides methods of screening for polypeptides with superior physicochemical properties to those of a polypeptide with predetermined physicochemical properties by using the polypeptide as a standard. More specifically, the present invention provides methods of screening for polypeptides with improved plasma kinetics, reduced immunogenicity, or improved solubility in comparison to a polypeptide with predetermined plasma kinetics, immunogenicity, or solubility, by using the extracellular matrix-binding activity of the polypeptides as an indicator. Furthermore, the present invention provides methods for producing polypeptides having antigen-binding activity that are specifically useful for use as pharmaceutical compositions, in particular, antibodies.

Specifically, the present invention provides a method of screening for polypeptides with superior physicochemical properties to those of a standard polypeptide, by using a polypeptide with predetermined physicochemical properties as a standard polypeptide, wherein the method comprises the steps of:

(a) contacting the standard polypeptide and a test polypeptide or a test polypeptide library with an extracellular matrix;
(b) measuring the extracellular matrix-binding activities of the polypeptides contacted in step (a); and
(c) selecting from the polypeptides measured in step (b), a polypeptide with lower extracellular matrix-binding activity than that of the standard polypeptide.

Steps (a) to (c) may be repeated two times or more. The number of times steps (a) to (c) are repeated is not particularly limited, but it is usually ten times or less.

The polypeptides used in the production methods and screening methods of the present invention may be prepared in any way, and for example, pre-existing antibodies, pre-existing libraries (phage libraries and such), antibodies or libraries produced from hybridomas obtained from immunization of animals or B cells from immunized animals, and antibodies or libraries produced by randomly modifying the amino acids of these antibodies or libraries may be used.

Furthermore, polypeptides produced by the production methods of the present invention and polypeptides obtained by the screening methods of the present invention may be able to improve plasma kinetics and reduce immunogenicity when administered to animals such as humans, mice, and monkeys, and improve solubility when prepared as solution formulations. Therefore, the production methods of the present invention can be used as production methods or screening methods for polypeptides with improved plasma kinetics, reduced immunogenicity, or improved solubility.

Moreover, the polypeptides produced by the production methods of the present invention and polypeptides obtained by the screening methods of the present invention are polypeptides with improved plasma kinetics, reduced immunogenicity, or improved solubility in comparison to the polypeptides before the modification of extracellular matrix-binding activity. Thus, they may be particularly superior when used as pharmaceuticals. Therefore, the production methods of the present invention may be used as methods for producing polypeptides to be used as pharmaceutical compositions.

Furthermore, since the screening methods of the present invention enable easy selection of polypeptides having superior plasma kinetics, immunogenicity, and solubility by using extracellular matrix-binding activity as an indicator, they can be used to obtain polypeptides particularly suitable for pharmaceuticals.

DNAs encoding a polypeptide obtained by the production methods of the present invention or a polypeptide obtained by the screening methods of the present invention are typically carried by (inserted into) appropriate vectors, and then introduced into host cells. The vectors are not particularly limited as long as they stably retain the inserted nucleic acids such as DNAs. For example, when *E. coli* is used as the host, preferred cloning vectors include pBluescript vector (Stratagene); however, various commercially available vectors may be used. When using vectors to produce the polypeptides of the present invention, expression vectors are particularly useful. The expression vectors are not particularly limited as long as the vectors express the polypeptides *in vitro,* in *E. coli,* in culture cells, or in a body of an organism. For example, pBEST vector (Promega) is preferred for *in vitro* expression; pET vector (Invitrogen) is preferred for E. coli; pME18S-FL3 vector (GenBank Accession No. AB009864) is preferred for culture cells; and pME18S vector (Mol Cell Biol. (1988) 8: 466-472) is preferred for bodies of organisms. DNAs of the present invention can be inserted into the vectors by conventional methods, for example, by ligase reactions using restriction enzyme sites (Current protocols in Molecular Biology, edit. Ausubel et al., (1987) Publish. John Wiley & Sons, Section 11.4-11.11).

The above host cells are not particularly limited, and various host cells may be used depending on the purpose. Examples of cells for expressing the polypeptides include bacterial cells (for example, *Streptococcus, Staphylococcus, E. coli, Streptomyces,* and *Bacillus subtilis*), fungal cells (for example, yeast and *Aspergillus*), insect cells (for example, Drosophila S2 and Spodoptera SF9), animal cells (for example, CHO, COS, HeLa, C127, 3T3, BHK, HEK293, and Bowes melanoma cells), and plant cells. Vectors can be introduced into a host cell by known methods, for example, calcium phosphate precipitation methods, electroporation methods (Current protocols in Molecular Biology edit. Ausubel et al. (1987) Publish. John Wiley & Sons, Section 9.1-9.9), lipofection methods, and microinjection methods.

The host cells can be cultured by known methods. For example, when using animal cells as a host, DMEM, MEM, RPMI1640, or IMDM may be used as the culture medium. They may be used with serum supplements such as FBS or fetal calf serum (FCS) or may be cultured in serum-free cultures. The preferred pH is about 6 to 8 during the course of culturing. Culture is carried out typically at about 30 to 40 °C for about 15 to 200 hours. Medium is exchanged, aerated, or agitated, as necessary.

Appropriate secretion signals may be incorporated to the polypeptides of interest so that the polypeptides expressed in the host cell are secreted into the lumen of the endoplasmic reticulum, into the periplasmic space, or into the extracellular environment. These signals may be endogenous to the polypeptides of interest or may be heterologous signals.

On the other hand, for example, production systems using animals or plants may be used as systems for producing polypeptides *in vivo.* A DNA encoding a polypeptide of interest is introduced into an animal or plant and the polypeptide is produced in the body of the animal or plant, and then collected. The "hosts" of the present invention include such animals and plants.

The production system using animals include those using mammals or insects. It is possible to use mammals such as goats, pigs, sheep, mice, and bovines (Vicki Glaser, SPECTRUM Biotechnology Applications (1993)). The mammals may be transgenic animals.

For example, a DNA encoding a polypeptide of the present invention is prepared as a fusion gene with a DNA encoding a polypeptide specifically produced in milk, such as the goat beta-casein. Next, goat embryos are injected with DNA fragments containing the fusion gene, and then transplanted to female goats. Desired polypeptides can be obtained from milk produced by the transgenic goats, which are born from the goats that received the embryos, or from their offspring. Hormones may be administered as appropriate to increase the volume of milk containing the polypeptide produced by the transgenic goats (Ebert et al., Bio/Technology (1994) 12: 699-702).

Insects such as silkworms may be used to produce the polypeptides of the present invention. When silkworms are used, baculoviruses carrying a DNA encoding a polypeptide of interest can be used to infect silkworms, and the polypeptide of interest can be obtained from their body fluids.

Furthermore, when plants are used to produce the polypeptides of the present invention, for example, tobacco may be used. When tobacco is used, a DNA encoding a polypeptide of interest is inserted into a plant expression vector, for example, pMON 530, and then the vector is introduced into bacteria, such as *Agrobacterium tumefaciens.* The bacteria are then allowed to infect tobacco such as *Nicotiana tabacum,* and the desired polypeptides can be obtained from their leaves (Ma et al., Eur. J. Immunol. (1994) 24: 131-138). Alternatively, it is possible to infect duckweed (Lemna minor) with similar bacteria. After cloning, the desired polypeptides can be obtained from the duckweed cells (Cox KM et al., Nat. Biotechnol. 2006 Dec; 24(12): 1591-1597).

The thus obtained polypeptides may be isolated from the inside or outside (such as the medium and milk) of host cells, and purified as substantially pure and homogenous polypeptides. The methods for isolating and purifying polypeptides are not particularly limited, and isolation and purification methods usually used for polypeptide purification can be used. Polypeptides may be isolated and purified, by appropriately selecting and combining, for example, chromatographic columns, filtration, ultrafiltration, salting out, solvent precipitation, solvent extraction, distillation, immunoprecipitation, SDS-polyacrylamide gel electrophoresis, isoelectric focusing, dialysis, and recrystallization.

Chromatography includes, for example, affinity chromatography, ion exchange chromatography, hydrophobic chromatography, gel filtration, reverse-phase chromatography, and adsorption chromatography (Strategies for Protein Purification and Characterization: A Laboratory Course Manual. Ed Daniel R. Marshak et al., (1996) Cold Spring Harbor Laboratory Press). These chromatographies can be conducted using liquid phase chromatography such as HPLC and FPLC. Columns used for affinity chromatography include, protein A columns and protein G columns. Columns using protein A include, for example, Hyper D, POROS, and Sepharose F. F. (Pharmacia).

If needed, a polypeptide can be modified arbitrarily, and peptides can be partially deleted by allowing an appropriate protein modification enzyme to act before or after purification of the polypeptide. Such protein modification enzymes include, for example, trypsin, chymotrypsin, lysyl endopeptidases, protein kinases, and glucosidases.

The present invention also relates to pharmaceutical compositions that include polypeptides obtained by the production methods or the screening methods of the present invention. The polypeptides of the present invention, the polypeptides produced by the production methods of the present invention, and the polypeptides obtained by the screening methods of the present invention may be able to improve plasma kinetics or reduce immunogenicity by administration as compared to typical antibodies or to improve solubility when prepared as soluble formulations, and are therefore useful as pharmaceutical compositions. The pharmaceutical compositions of the present invention may include pharmaceutically acceptable carriers.

In the present invention, pharmaceutical compositions ordinarily refer to agents for treating or preventing, or testing and diagnosing diseases.

The pharmaceutical compositions of the present invention can be formulated by methods known to those skilled in the art. For example, they can be used parenterally, in the form of injections of sterile solutions or suspensions including water or other pharmaceutically acceptable liquid. For example, such compositions may be formulated by mixing in the form of unit dose required in the generally approved medicine manufacturing practice by appropriately combining with pharmaceutically acceptable carriers or media, specifically with sterile water, physiological saline, vegetable oil, emulsifier, suspension, surfactant, stabilizer, flavoring agent, excipient, vehicle, preservative, binder, or such. In such formulations, the amount of active ingredient is adjusted to obtain an appropriate amount in a pre-determined range.

Sterile compositions for injection can be formulated using vehicles such as distilled water for injection, according to standard formulation practice. Aqueous solutions for injection include, for example, physiological saline and isotonic solutions containing glucose or other adjuvants (for example, D-sorbitol, D-mannose, D-mannitol, and sodium chloride). It is also possible to use in combination appropriate solubilizers, for example, alcohols (ethanol and such), polyalcohols (propylene glycol, polyethylene glycol, and such), non-ionic surfactants (polysorbate 80(TM), HCO-50, and such).

Oils include sesame oil and soybean oils. Benzyl benzoate and/or benzyl alcohol can be used in combination as solubilizers. It is also possible to combine buffers (for example, phosphate buffer and sodium acetate buffer), soothing agents (for example, procaine hydrochloride), stabilizers (for example, benzyl alcohol and phenol), and antioxidants. Generally, appropriate ampules are filled with the prepared injections.

The pharmaceutical compositions of the present invention are preferably administered parenterally. For example, the compositions may be in the dosage form for injections, transnasal administration, transpulmonary administration, or transdermal administration. For example, they can be administered systemically or locally by intravenous injection, intramuscular injection, intraperitoneal injection, subcutaneous injection, or such.

Administration methods can be appropriately selected in consideration of the patient's age and symptoms. The dose of a pharmaceutical composition containing a polypeptide of the present invention may be, for example, from 0.0001 to 1,000 mg/kg for each administration. Alternatively, the dose may be, for example, from 0.001 to 100,000 mg per patient. However, the present invention is not limited by the numeric values described above. The doses and administration methods vary depending on the patient's weight, age, symptoms, and such. Those skilled in the art can set appropriate doses and administration methods in consideration of the factors described above. The pharmaceutical compositions of the present invention can be pharmaceutical compositions that are used for improving plasma kinetics or reducing immunogenicity, or for improving solubility when prepared as solution formulations.

Moreover, the present invention relates to methods for improving plasma kinetics or methods for reducing immunogenicity by administering into subjects the polypeptides of the present invention, polypeptides produced by the production methods of the present invention, or the above-described pharmaceutical compositions of the present invention. Administration may be carried out *in vivo* or *in vitro.* Subjects of administration include, for example, non-human animals (such as mice and monkeys) and humans.

Amino acids contained in the amino acid sequences of the present invention may be post-translationally modified (for example, the modification of an N-terminal glutamine into a pyroglutamic acid by pyroglutamylation is well-known to those skilled in the art). Naturally, amino acid sequences with such post-translationally modified amino acids are also included in the amino acid sequences in the present invention.

All prior art documents cited in the specification are incorporated herein by reference.

Examples

Herein below, the present invention will be specifically described with reference to the Examples, but it is not to be construed as being limited thereto.

[Example 1] Evaluation of the relationship between extracellular matrix binding and plasma pharmacokinetics in normal mice

MabA1, A2, and A3 are bispecific antibodies that show specific binding to blood coagulation factor IX and/or activated blood coagulation factor IX and blood coagulation factor X as target antigens, and exhibit blood coagulation factor VIII-like coagulation activity. These antibodies are IgG antibodies obtained by using the methods described in WO2005/035756 and WO2006/109592, performing screening using the target antigen binding and the coagulation activity as indicators based on techniques known to those skilled in the art for obtaining antibodies, and adding amino acid modifications that improve the blood coagulation factor VIII-like coagulation activity.

To compare the retention of MabA1, A2, and A3 in plasma, pharmacokinetics of these antibodies in mice were evaluated.

MabA1, A2, and A3 were given to normal mice (C57BL/6J, Charles River Laboratories Japan) in a single administration at 1 mg/kg, and blood was collected when appropriate. The collected blood was immediately centrifuged at 4°C for 15 minutes at 15,000 rpm to obtain blood plasma. The separated plasma was stored in a freezer set at -20°C or lower until measurement was performed.

Measurement of concentration in mouse plasma was carried out using the ELISA method. First, anti-human IgG(γ) was dispensed into a 96-well plate, and left to stand overnight at 4°C to prepare an anti-human IgG(γ)-immobilized plate. Samples for standard curve and samples for measuring mouse plasma were prepared. They were dispensed into the anti-human IgG(γ)-immobilized plate, and left to stand for one hour at room temperature. Then, these samples were reacted with anti-human IgG-AP (manufactured by SIGMA), color development reaction was carried out using the Blue-Phos Microwell Phosphatase Substrates System (manufactured by Kirkegaard & Perry Laboratories) as substrate, and absorbance at 650 nm was measured on a microplate reader. The concentration in mouse plasma was derived from the absorbance in the standard curve using the analysis software SOFTmax PRO (manufactured by Molecular Devices).

The plasma half-lives of MabA1, A2, and A3 in normal mice were 17.1 days, 6.1 days, and 1.9 days, respectively, and they were found to differ greatly. This difference in plasma retention (plasma half-life) may be due to non-specific extracellular matrix binding. More specifically, antibodies showing extracellular matrix-binding ability may have been disappearing quickly as a result of *in vivo* extracellular matrix binding in the plasma.

Therefore, the extracellular matrix-binding abilities of MabA1, A2, and A3 were evaluated. Extracellular matrix binding of the antibodies was measured using the ELISA method. Blocking was performed by dispensing a blocking solution [TBS (TaKaRa), 10% FCS (Low IgG / manufactured by GIBCO), 0.05% NaN₃] onto an extracellular matrix-immobilized plate (Matrigel Matrix Thin Layer 96-well plate / manufactured by BD). The blocking solution was removed from the cell membrane matrix-immobilized plate. Samples prepared by diluting various antibodies with the blocking solution were dispensed, left at room temperature for one hour, and binding reactions between the extracellular matrix and various antibodies were carried out. The various antibody samples were removed from the plate, the plate was washed three times using a rinsing buffer [Dulbecco's PBS (Nissui), 0.05% Tween 20], and Goat Anti-human IgG-HRP (manufactured by Zymed-Invitrogen) was allowed to react at room temperature for one hour. After the reaction, the plate was washed five times using the rinsing buffer, and color development reaction was performed using the ABTS Microwell Peroxidase Substrate (2-Component System/manufactured by KPL) as substrate. The reaction was terminated by the addition of 1 M $H_2SO_4$ solution, and absorbance at 405 nm was measured on a microplate reader.

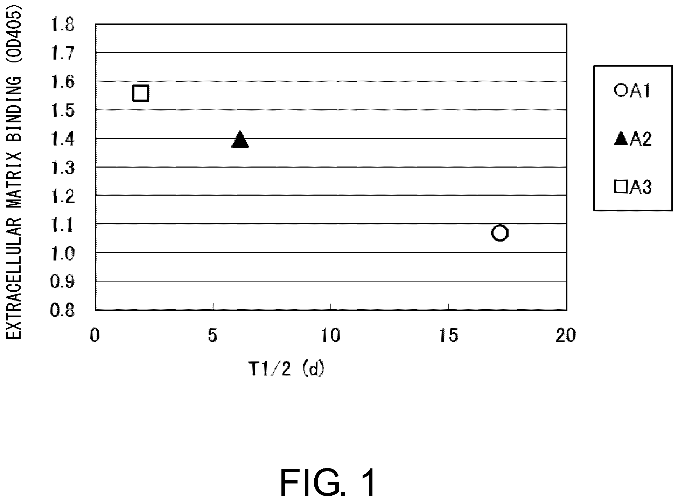

The extracellular matrix binding of MabA1, A2, and A3 is plotted against plasma half-life (T1/2) in Fig. 1. As a result, antibodies having low extracellular matrix-binding ability maintained a high plasma antibody concentration and a long plasma half-life, and it became clear that they have superior pharmacokinetics. These results showed that extracellular matrix binding and plasma half-life (T1/2) are correlated, and suggested that measurement of extracellular matrix binding is a suitable method for predicting plasma pharmacokinetics, and lowering the extracellular matrix-binding ability of antibodies can improve their plasma pharmacokinetics (plasma half-lives).

[Example 2] Increasing the sensitivity of detection of extracellular matrix binding and improving plasma retention of MabB1 by amino acid substitutions

MabB1 is an IgG antibody produced by a method similar to Example 1 by performing amino acid substitutions that increase the blood coagulation factor VIII-like coagulation activity on an antibody obtained by screening using as indices the blood coagulation factor VIII-like coagulation activity and the binding to both target antigens, i.e., blood coagulation factor IX and/or activated blood coagulation factor IX as well as blood coagulation factor X, by methods known to those skilled in the art. MabB1 has a very short plasma half-life, and this is an important issue when developing MabB1 into a pharmaceutical, since short plasma half-life leads to increase in dose and shortening of the interval between administrations. Evaluation of the binding of MabB1 to extracellular matrix confirmed that MabB1 binds strongly to the extracellular matrix, and the reason for short plasma half-life may be due to non-specific binding to extracellular matrix. Therefore, non-specific binding to extracellular matrix was lowered, and improvement of plasma pharmacokinetics of MabB1 was examined.

Since MabB1 has high extracellular matrix-binding ability, various modified MabB1 antibodies were produced by methods known to those skilled in the art by introducing amino acid substitutions into surface-exposed residues in the antibody variable region of MabB1, and they were screened for extracellular matrix binding. Of the various modified MabB1 antibodies, MabB2 and MabB3 had reduced extracellular matrix-binding ability and were obtained.

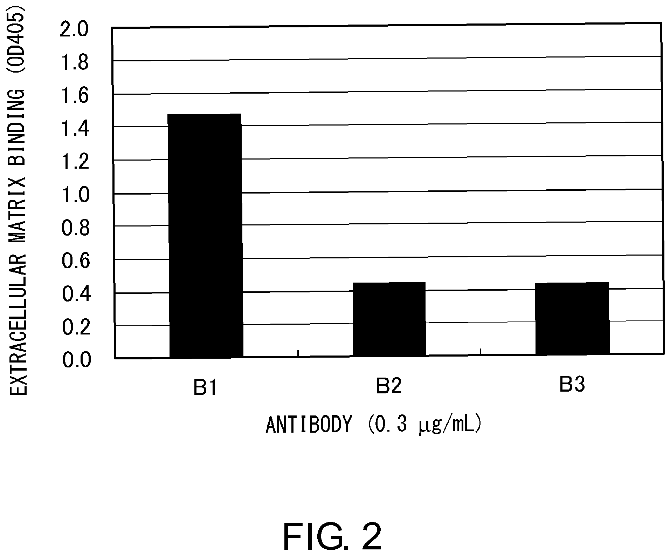

The results of measuring the extracellular matrix binding of the antibodies by the above-mentioned colorimetric ELISA method are shown in Fig. 2. While strong extracellular matrix binding was observed with MabB 1, extracellular matrix binding could not be observed with MabB2 and MabB3 into which amino acid substitutions have been introduced.

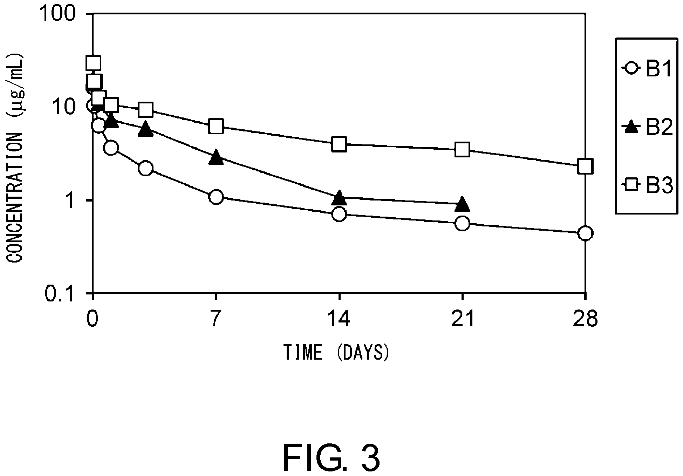

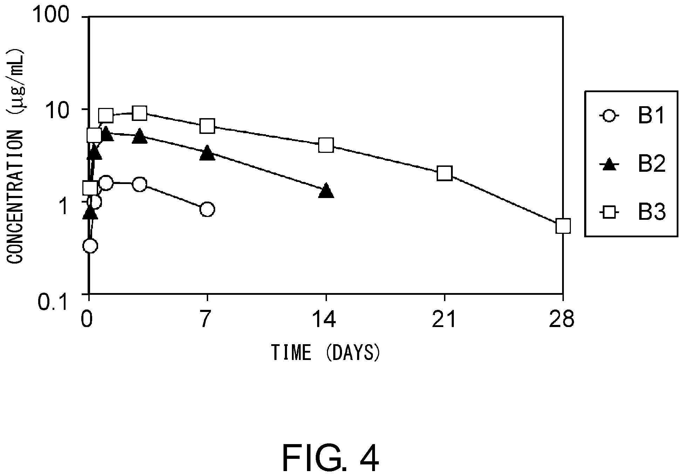

The results of evaluating the pharmacokinetics of each antibody (MabB1 to B3) in normal mice by the method demonstrated in Example 1 are shown in Fig. 3 (intravenous administration) and Fig. 4 (subcutaneous administration). As a result, plasma retention increased in both intravenous administration and subcutaneous administration of MabB2 and MabB3 in comparison to MabB1. Indeed it was confirmed that the plasma retention of the antibodies can be improved by decreasing their extracellular matrix-binding ability. However, when compared to MabB2, MabB3 had better plasma retention, suggesting the possibility that MabB2 still has extracellular matrix-binding ability.

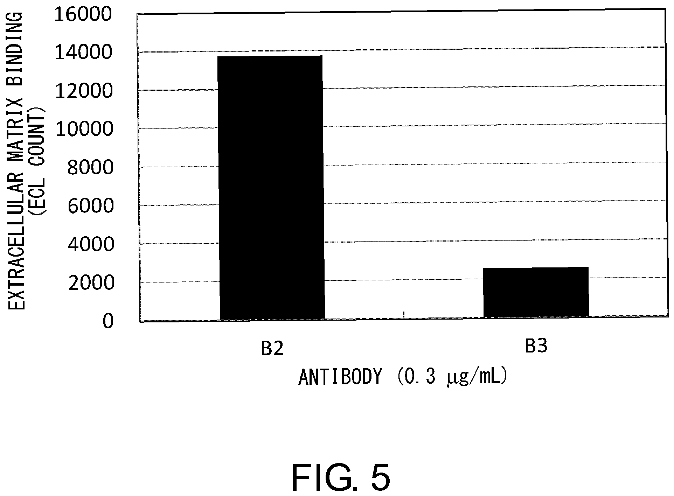

Since the extracellular matrix-binding ability of MabB2 could not be observed by the ELISA method, the detection sensitivity of the evaluation system using ELISA was considered to be insufficient. Accordingly, there was an attempt for the following method of using electrochemiluminescence (ECL) to evaluate extracellular matrix binding with high sensitivity. An evaluation system that uses ECL to detect the extracellular matrix-binding ability of antibodies with higher sensitivity was established. The extracellular matrix (BD Matrigel Basement Membrane Matrix / manufactured by BD) was diluted to 5 mg/mL using a solution of TBS (Takara) containing 0.015% Triton-XlOO. The diluted extracellular matrix was dispensed into an uncoated MULTI-ARRAY PR Plate (manufactured by MSD), and fixed overnight at 4°C. Subsequently, a solution of Dulbecco's PBS (Nissui) containing 0.05% Tween 20, 0.5% BSA, and 0.01% $NaN_3$ was dispensed into the plate for blocking. Goat anti-human IgG (gamma) (Zymed Laboratories) was Sulfo-Tagged using 150 nmol of Sulfo-Tag NHS Ester (manufactured by MSD). Various antibodies and Sulfo-Tagged Goat anti-human IgG were mixed well and incubated at room temperature for about one hour. This sample was dispensed into the extracellular matrix-immobilized plate after removal of the blocking solution, and this was shaken at room temperature for two hours. Subsequently, MSD Read Buffer T (4x) (manufactured by MSD) was added, and the amount of luminescence was measured on SECTOR PR400. Results of measuring the extracellular matrix binding of antibodies (MabB2 and MabB3) by the above-mentioned method are shown in Fig. 5. While extracellular matrix binding of MabB2 could not be detected by ELISA (Fig. 2), it could be confirmed by the highly sensitive system using ECL. That is, the use of ECL showed that extracellular matrix binding of the three antibodies, MabB1, MabB2, and MabB3, correlates with their plasma retention.

This led to the discovery that antibodies with improved plasma pharmacokinetics can be obtained by screening antibodies produced by introducing modifications such as amino acid substitutions into antibodies, with the use of a highly sensitive extracellular matrix binding evaluation system that uses ECL.

[Example 3] Improving plasma retention of MabC9 by reducing its extracellular matrix binding

MabC9 is an IgG antibody produced by a method similar to Example 1 by performing amino acid substitutions that increase the blood coagulation factor VIII-like coagulation activity on an antibody obtained by screening using as indices the blood coagulation factor VIII-like coagulation activity and the binding to both target antigens, i.e., blood coagulation factor IX and/or activated blood coagulation factor IX as well as blood coagulation factor X, using methods known to those skilled in the art. MabC9 was known to have a very short plasma half-life, and therefore improvement of the plasma pharmacokinetics of MabC9 was examined. The results confirmed that MabC9 strongly binds to extracellular matrix, and the reason for the short plasma half-life was considered to be non-specific extracellular matrix binding.

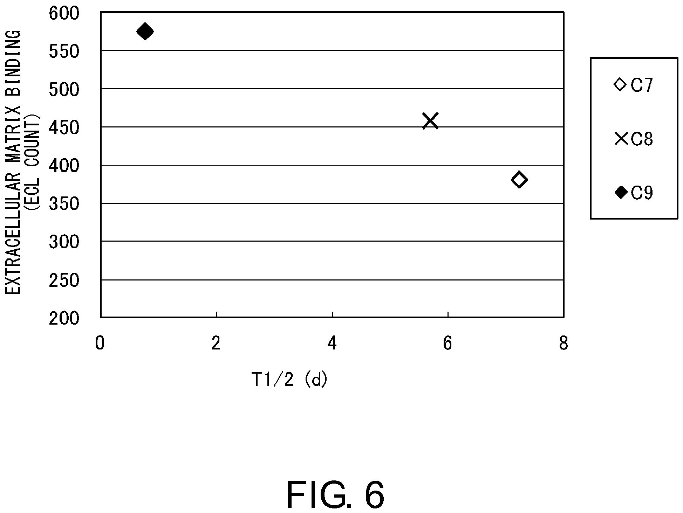

Since MabC9 has high extracellular matrix-binding ability, various modified MabC9 antibodies were produced by methods known to those skilled in the art by introducing amino acid substitutions into the surface-exposed residues in the antibody variable region of MabC9, and they were screened for extracellular matrix binding. Of the various modified MabC9 antibodies, MabC8 and MabC7 had reduced extracellular matrix-binding abilities and they were obtained. MabC9, MabC8, and MabC7 were administered intravenously at 1 mg/kg to human FcRn transgenic mice (B6.mFcRn-/-.hFcRn Tg line 276 +/+ mice, Jackson Laboratories), and their plasma pharmacokinetics was evaluated. As a result, as shown in Fig. 6, MabC8 and MabC7 which have reduced extracellular matrix-binding abilities showed greatly prolonged plasma half-lives as compared to MabC9, and their pharmacokinetics was found to be improved.

This led to the discovery that the plasma pharmacokinetics of an antibody can be improved by introducing modifications such as amino acid substitutions into the antibody and lowering its extracellular matrix-binding ability.

[Example 4] Evaluation of the relationship between extracellular matrix binding and immunogenicity

MabC1 to C6 are IgG antibodies produced by a method similar to Example 1 by performing amino acid substitutions that increase the blood coagulation factor VIII-like coagulation activity on an antibody obtained by screening using as indices the blood coagulation factor VIII-like coagulation activity and the binding to both target antigens, i.e., blood coagulation factor IX and/or activated blood coagulation factor IX as well as blood coagulation factor X, using methods known to those skilled in the art. As a result of evaluating the extracellular matrix binding of MabC1 to C6 by ECL, the degrees of extracellular matrix binding of these antibodies were found to be different depending on the antibody. Since antibodies that bind to an extracellular matrix show non-specific binding to non-target antigens, they may also bind to antigen-presenting cells. Therefore, antibodies that bind to an extracellular matrix are thought to be easily taken up by antigen-presenting cells and consequently easily presented as antigens, leading to higher immunogenicity. Immunogenicity is an important issue in protein pharmaceuticals, and the immunogenicity of a protein pharmaceutical is desirably low.

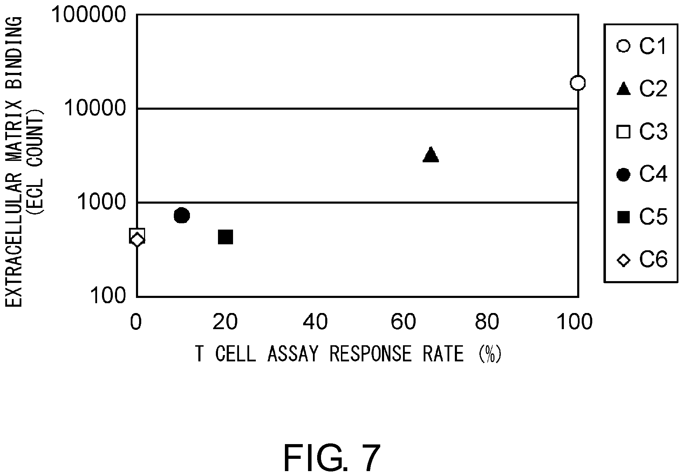

Therefore, to assess the risk of immunogenicity induced against each of the antibodies (C1 to C6) having different extracellular matrix-binding abilities, *in vitro* immunogenicity risk assessment was carried out using fresh blood of volunteers. CD8-negative CD25-weakly positive peripheral blood mononuclear cells (CD8- CD25low PBMCs) separated from fresh human blood were seeded in 24-well plates, and pre-cultured for approximately two hours at 37°C in 5% $CO_2$ under humid conditions. After pre-culturing, antibodies were added and the cells were cultured at 37°C in 5% $CO_2$ under humid conditions. Cells collected on days 6, 7, and 8 of culturing were seeded again in 96-well plates, and bromodeoxyuridine (BrdU) was added. Approximately 30 hours after the addition, Helper T cells that have taken up BrdU were analyzed by flow cytometry. The degree of proliferation activity against each antibody was examined based on the proportion of BrdU-positive cells in the Helper T cells. The experiment was performed in several donors, and each of the antibodies was evaluated for the percentage of donors showing proliferation activity. Higher percentage of responding donors suggests higher immunogenicity. Fig. 7 shows a plot in which the horizontal axis represents the response rate of donors showing proliferation activity, and the vertical axis represents extracellular matrix binding.

As a result, the extracellular matrix binding of each antibody and the response rate of donors showing proliferation activity showed a positive correlation, such that the lower the extracellular matrix-binding activity, the lower the immunogenicity. This suggests that the immunogenicity of an antibody can be reduced by lowering its extracellular matrix-binding activity.

[Example 5] Decrease of MabB2 immunogenicity due to decrease of extracellular matrix binding

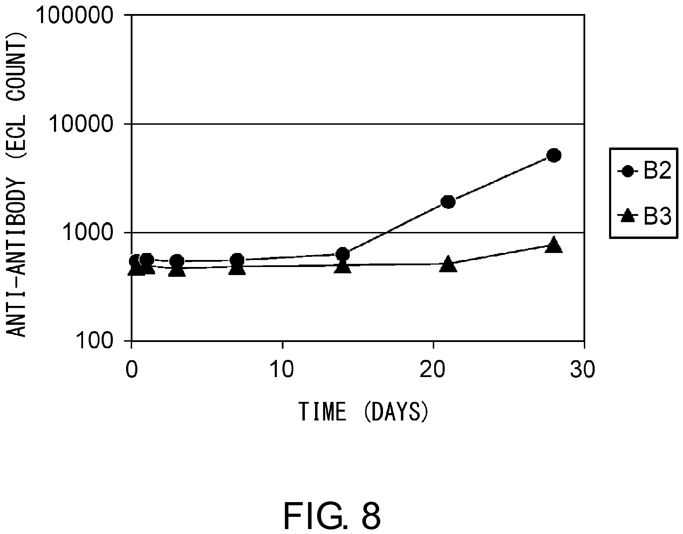

When MabB2 produced in Example 2 is administered to mice, anti-MabB2 mouse antibodies are produced in a short period of time, and thus it is confirmed that MabB2 has high immunogenicity. In Example 4, antibodies with lower extracellular matrix-binding ability had lower immunogenicity, and this suggests that the immunogenicity of antibodies can be decreased by reducing their extracellular matrix-binding ability. Therefore, to evaluate the plasma retention of MabB2 and MabB3 in mice, anti-MabB2 antibodies and anti-MabB3 antibodies in mouse plasma were detected by ECL immunoassay. First, antibodies were biotinylated using EZ-Link™ Sulfo-NFS-Biotinylation Kit (manufactured by PIERCE), and sTagged using 150 nmol of Sulfo-Tag NHS Ester (manufactured by MSD). A sample for mouse plasma measurement was mixed with biotinylated antibodies and sTagged antibodies, and then left to stand overnight at 4°C. This sample was dispensed into a MULTI-ARRAY(R) PR SA Plate (manufactured by MSD), and the plate was shaken at room temperature for two hours. Then, MSD Read Buffer T (4x) (manufactured by MSD) was added, and the amount of luminescence was measured on SECTOR PR(R) 400. Fig. 8 shows a plot in which the horizontal axis represents elapsed time (days), and the vertical axis represents antibody titer (ECL signal). In comparison to MabB2, MabB3 which had a reduced extracellular matrix-binding ability was confirmed to lead to a lower antibody titer and to have reduced immunogenicity.

Up to now, as methods for decreasing antibody immunogenicity, methods of humanizing non-human antibodies, methods of removing T-cell epitopes, and methods of decreasing aggregates are known. However, this led to the discovery that the immunogenicity of an antibody can be decreased by introducing modifications such as amino acid substitutions into the antibody and reducing its extracellular matrix-binding ability.

[Example 6] Evaluation of the relationship between extracellular matrix binding and solubility

MabD1 to D30 are IgG antibodies produced by a method similar to Example 1 by performing amino acid substitutions that increase the blood coagulation factor VIII-like coagulation activity on an antibody obtained by screening using as indices the blood coagulation factor VIII-like coagulation activity and the binding to both target antigens, i.e., blood coagulation factor IX and/or activated blood coagulation factor IX as well as blood coagulation factor X, using methods known to those skilled in the art. As a result of evaluating the extracellular matrix binding of MabD1 to D3 by ECL, the degrees of extracellular matrix binding of these antibodies were found to be different depending on the antibody. To prepare a solution formulation of a protein pharmaceutical, it is necessary that the protein has sufficiently high solubility. When proteins easily self-associate (aggregate) with each other, their solubility is generally considered to be low. It is thought that antibodies that bind to the extracellular matrix have the property of easily binding to proteins non-specifically, and their solubility is likely to be low. Solubility is important for producing solution formulations of protein pharmaceuticals, and it is desirable that protein pharmaceuticals have high solubility.

Accordingly, the relationship between extracellular matrix binding and solubility of MabD1 to D30 was evaluated. Generally, large quantities of proteins are needed for measuring protein solubility; and while protein solubility decreases greatly in the presence of polyethylene glycol (PEG), it is possible to measure solubility using a small quantity of protein by measuring solubility in a buffer in the presence of a certain concentration of PEG (Guiotto, A. et al. (2004) Bioorg. Med. Chem. 12, 5031-5037). Accordingly, the solubility of various antibodies (MabD1 to MabD30) was measured in the presence of PEG. To 95 μL each of a solution of 10 mM sodium phosphate containing 50 mM NaCl at pH 6 (buffer solution) and a solution of 10 mM sodium phosphate containing 50 mM NaCl at pH 6 with 10% PEG4000 (PEG solution), 5 μL of various antibody solutions with known concentrations were added and mixed well. After about three hours of incubation at room temperature, this was passed through a 0.22-μm filter, and the filtered solution (70 μL) was diluted two-fold by adding 70 μL of a solution of 50 mM sodium phosphate containing 500 mM NaCl at pH 7.0. The diluted solution (100 μL) was analyzed by gel filtration chromatography. Gel filtration chromatography was carried out under the following conditions:

    column: G3000SWxl (TOSOH);

mobile phase: 50 mM sodium phosphate, 500 mM NaCl, pH7.0;
flow rate: 1.0 mL/min; and
detection wavelength: 220 nm.

The areas of PEG solution monomer peak and buffer solution monomer were calculated for each antibody. The solubility of each antibody in the presence of PEG was calculated according to the following calculation method:

antibody solution concentration (mg/mL) x PEG solution monomer peak area / buffer solution monomer area

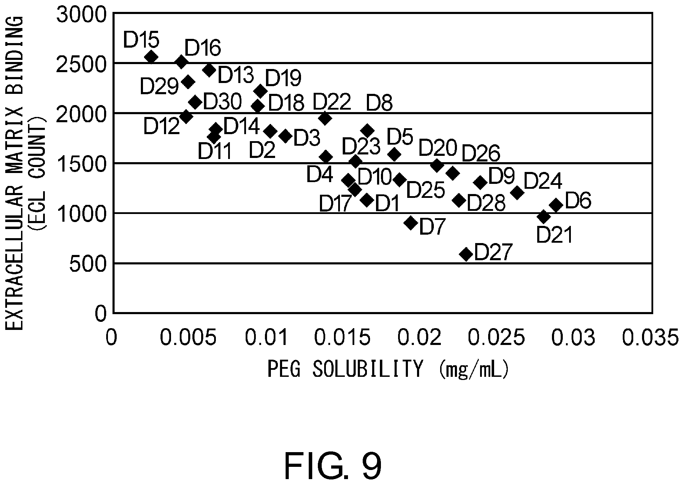

Fig. 9 shows a plot in which the horizontal axis represents extracellular matrix binding and the vertical axis represents solubility in the presence of PEG. It was discovered that there is a high correlation between extracellular matrix binding and solubility, and that the higher the extracellular matrix binding, the lower the solubility. More specifically, this showed that antibodies with high solubility can be obtained by screening for antibodies having low extracellular matrix binding.

[Example 7] Improvement of the solubility of MabD31 by decreasing its extracellular matrix binding

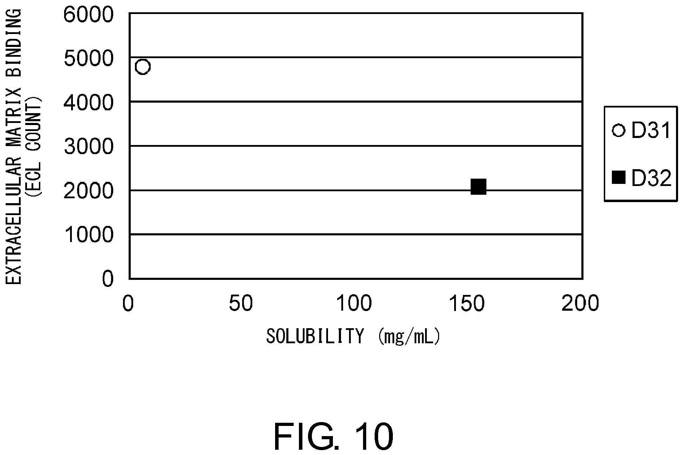

MabD31 is an IgG antibody produced by a method similar to Example 1 by performing amino acid substitutions that increase the blood coagulation factor VIII-like coagulation activity on an antibody obtained by screening using as indices the blood coagulation factor VIII-like coagulation activity and the binding to both target antigens, i.e., blood coagulation factor IX and/or activated blood coagulation factor IX as well as blood coagulation factor X, using methods known to those skilled in the art. MabD31 has been confirmed to have a low solubility. Example 6 shows that antibodies with high solubility can be obtained by screening for antibodies having low extracellular matrix binding. Therefore, various mutant MabD31 antibodies were produced against MabD31 by introducing amino acid substitutions into surface-exposed residues exposed in the MabD31 antibody variable region, and screening for extracellular matrix binding. MabD32 which has reduced extracellular matrix binding was produced by introducing amino acid substitutions into the variable region of MabD31. As a result of measuring the solubility of MabD31 and MabD32 under conditions of 20 mM histidine, 150 mM NaCl, and pH 6.0, the solubility of MabD31 was 5.9 mg/mL, whereas the solubility of MabD32 was 150 mg/mL or more. Fig. 10 shows the extracellular matrix binding and solubility of MabD31 and MabD32.
Consequently, it was found that the solubility of an antibody can be improved by decreasing its extracellular matrix binding.

Industrial Applicability

The present invention provides methods for improving the physicochemical properties of polypeptides and methods for producing polypeptides with improved physicochemical properties by lowering the extracellular matrix binding of polypeptides having functions of interest. In particular, improvement of physicochemical properties of the polypeptides may be improvement of plasma kinetics, reduction of immunogenicity, or improvement of solubility; and polypeptides suitable for use as pharmaceuticals can be provided. Furthermore, physicochemical properties of a test polypeptide can be evaluated by comparing the extracellular matrix-binding activity of the test polypeptide with the extracellular matrix-binding activity of a polypeptide having predetermined physicochemical properties.
The invention particularly also provides the following items 1 to 24:

1. A method for improving a physicochemical property of a polypeptide by reducing its extracellular matrix-binding activity.

2. The method of item 1, wherein the improvement of a physicochemical property is at least one selected from improvement of plasma kinetics, reduction of immunogenicity, and improvement of solubility.

3. The method of item 1 or 2, wherein reduction of the extracellular matrix-binding activity is accomplished by modifying at least one amino acid residue of the polypeptide.

4. The method of any one of items 1 to 3, wherein the extracellular matrix-binding activity is measured by an electrochemiluminescence method.

5. The method of any one of items 1 to 4, wherein the polypeptide is a polypeptide having antigen-binding activity.

6. The method of item 5, wherein the polypeptide having antigen-binding activity is an antibody.

7. A method for producing a polypeptide with improved physicochemical property, which comprises the step of modifying at least one amino acid residue of a polypeptide such that its extracellular matrix-binding activity is reduced.

8. A method for producing a polypeptide with improved physicochemical property, which comprises the steps of:

(a) modifying at least one amino acid residue of a polypeptide;
(b) measuring the extracellular matrix-binding activity of the polypeptide modified in step (a); and
(c) selecting a polypeptide whose extracellular matrix-binding activity is reduced in comparison to before the modification.

9. The production method of item 8, which further comprises the steps of:

(d) obtaining a gene encoding the polypeptide selected in step (c) of item 8; and
(e) producing a polypeptide using the gene obtained in step (d).

10. The production method of item 9, which further comprises the step of:
(f) modifying at least one amino acid residue of the polypeptide obtained in step (e) of item 9, and repeatedly performing steps (b) to (e) mentioned above.

11. A method for producing a polypeptide having a superior physicochemical property to that of a standard polypeptide by using a polypeptide having a predetermined physicochemical property as the standard polypeptide, wherein the method comprises the steps of:

(a) contacting the standard polypeptide and a test polypeptide or a test polypeptide library with an extracellular matrix;
(b) measuring the extracellular matrix-binding activity of the polypeptides contacted in step (a);
(c) selecting from the polypeptides measured in step (b), a polypeptide whose extracellular matrix-binding activity is lower than that of the standard polypeptide;
(d) obtaining a gene encoding the polypeptide selected in step (c); and
(e) producing a polypeptide using the gene obtained in step (d).

12. The production methods of items 7 to 11, wherein the improvement of a physicochemical property is at least one selected from improvement of plasma kinetics, reduction of immunogenicity, and improvement of solubility.

13. The production method of any one of items 7 to 12, wherein the extracellular matrix-binding activity is measured by an electrochemiluminescence method.

14. The production method of any one of items 7 to 13, wherein the polypeptide is a polypeptide having antigen-binding activity.

15. The production method of item 14, wherein the polypeptide having antigen-binding activity is an antibody.

16. A polypeptide produced by the production method of any one of items 7 to 15.

17. A method of screening for a polypeptide having a superior physicochemical property to that of a standard polypeptide by using a polypeptide having a predetermined physicochemical property as the standard polypeptide, wherein the method comprises the steps of:

(a) contacting the standard polypeptide and a test polypeptide or a test polypeptide library with an extracellular matrix;
(b) measuring the extracellular matrix-binding activity of the polypeptides contacted in step (a); and
(c) selecting from the polypeptides measured in step (b), a polypeptide whose extracellular matrix-binding activity is lower than that of the standard polypeptide.

18. The screening method of item 17, wherein the improvement of a physicochemical property is at least one selected from improvement of plasma kinetics, reduction of immunogenicity, and improvement of solubility.

19. The screening method of item 17 or 18, wherein the extracellular matrix-binding activity is measured by an electrochemiluminescence method.

20. The screening method of any one of items 17 to 19, wherein the polypeptide is a polypeptide having antigen-binding activity.

21. The screening method of item 20, wherein the polypeptide having antigen-binding activity is an antibody.

22. A method for evaluating a physicochemical property of a test polypeptide by measuring the extracellular matrix-binding of the test polypeptide and comparing it to the extracellular matrix-binding activity of another polypeptide whose physicochemical property is predetermined.

23. The method of item 22, wherein the physicochemical property is at least one selected from plasma kinetics, immunogenicity, and solubility of the polypeptide.

24. The method of item 22 or 23, wherein measurement of the extracellular matrix-binding activity is measurement by an electrochemiluminescence method.

**Claims**

1. A method for producing an antibody with improved physicochemical property, wherein the improvement of a physicochemical property is at least one selected from improvement of plasma kinetics, reduction of immunogenicity, and improvement of solubility, which method comprises

> i) the step of modifying at least one amino acid residue of an antibody such that its extracellular matrix-binding activity is reduced,
> or
> ii) the steps of:
>
> > (a) modifying at least one amino acid residue of an antibody;
> > (b) measuring the extracellular matrix-binding activity of the antibody modified in step (a); and
> > (c) selecting an antibody whose extracellular matrix-binding activity is reduced in comparison to before the modification.

2. The production method of claim 1, alternative ii),
   wherein steps (a) and (b) are optionally repeated two times or more,
   and/or
   wherein the method further comprises the steps of:

> (d) obtaining a gene encoding the antibody selected in step (c); and
> (e) producing an antibody using the gene obtained in step (d),
> particularly wherein the production method further comprises the step of:
> (f) modifying at least one amino acid residue of the antibody obtained in step (e), and repeatedly performing steps (b) to (e) mentioned above.

3. The production method of claim 1 or 2, wherein at least one amino acid residue located on the surface of said antibody is modified, especially wherein variable-region amino acids exposed on the antibody surface are modified.

4. A method for producing an antibody having a superior physicochemical property to that of a standard antibody by using an antibody having a predetermined physicochemical property as the standard antibody, wherein the method comprises the steps of:

> (a) contacting the standard antibody and a test antibody or a test antibody library with an extracellular matrix;
> (b) measuring the extracellular matrix-binding activity of the antibodies contacted in step (a);
> (c) selecting from the antibodies measured in step (b), an antibody whose extracellular matrix-binding activity is lower than that of the standard antibody;
> (d) obtaining a gene encoding the antibody selected in step (c); and

(e) producing an antibody using the gene obtained in step (d).

5. The production method of claim 4,

   i) wherein at least one amino acid residue of said standard antibody, particularly at least one amino acid residue located on the surface of said standard antibody, has been modified in said test antibody, especially wherein variable-region amino acids exposed on the antibody surface have been modified,
   and/or
   ii) wherein steps (a) to (c) are optionally repeated two times or more.

6. A method for producing an antibody, wherein

   (a) at least one amino acid residue of the antibody has been modified;
   (b) the extracellular matrix-binding activity of the polypeptide modified in step (a) has been measured; and
   (c) the antibody is selected as an antibody whose extracellular matrix-binding activity is reduced in comparison to before the modification.

7. The method of claim 6, wherein

   i) said method is a method for producing an IgG antibody, wherein

      (a') at least one amino acid residue of the IgG antibody has been substituted;
      (b') the non-specific binding activity of the IgG antibody for one or more extracellular matrix proteins has been measured by an electrochemiluminescence method, wherein said one or more extracellular matrix proteins are selected from the group consisting of collagen, proteoglycans, fibronectin, laminin and entactin;
      (c') the IgG antibody is selected as an IgG antibody whose non-specific binding activity to said one or more extracellular matrix proteins is reduced in comparison to before the substitution,

   and/or
   ii) at least one amino acid residue located on the surface of said antibody has been modified, especially wherein variable-region amino acids exposed on the antibody surface have been modified.

8. A method for improving a physicochemical property of an antibody by reducing its extracellular matrix-binding activity, wherein the improvement of a physicochemical property is at least one selected from improvement of plasma kinetics, reduction of immunogenicity, and improvement of solubility, and wherein reduction of the extracellular matrix-binding activity is accomplished by modifying at least one amino acid residue of the antibody.

9. The method of claim 8, wherein at least one amino acid residue located on the surface of said antibody is modified, especially wherein variable-region amino acids exposed on the antibody surface are modified.

10. A method of screening for an antibody having a superior physicochemical property to that of a standard antibody by using an antibody having a predetermined physicochemical property as the standard antibody, wherein the improvement of a physicochemical property is at least one selected from improvement of plasma kinetics, reduction of immunogenicity, and improvement of solubility, and wherein the method comprises the steps of:

   (a) contacting the standard antibody and a test antibody or a test antibody library with an extracellular matrix;
   (b) measuring the extracellular matrix-binding activity of the antibodies contacted in step (a); and
   (c) selecting from the antibodies measured in step (b), an antibody whose extracellular matrix-binding activity is lower than that of the standard antibody.

11. The method of claim 10,

   i) wherein at least one amino acid residue of said standard antibody, particularly at least one amino acid residue located on the surface of said standard antibody, has been modified in said test antibody, especially wherein variable-region amino acids exposed on the antibody surface have been modified,
   and/or
   ii) wherein steps (a) and (b) are optionally repeated two times or more.

12. A method for evaluating a physicochemical property of a test antibody by measuring the extracellular matrix-binding of the test antibody and comparing it to the extracellular matrix-binding activity of another antibody whose physicochemical property is predetermined, wherein the physicochemical property is at least one selected from plasma kinetics, immunogenicity, and solubility of the antibody.

13. The method of claim 12, wherein at least one amino acid residue located on the surface of said other antibody has been modified in said test antibody, especially wherein variable-region amino acids exposed on the antibody surface have been modified.

14. The method of any of the preceding claims, wherein said extracellular matrix contains glycoproteins, particularly wherein said extracellular matrix contains glycoproteins such as collagen, proteoglycans, fibronectin, laminin, entactin, fibrin, and perlecan or wherein said extracellular matrix contains glycoproteins selected from collagen, proteoglycans, fibronectin, laminin, entactin, fibrin, and perlecan, especially wherein the extracellular matrix-binding activity is further defined as extracellular matrix-binding activity to one or more glycoproteins selected from collagen, proteoglycans, fibronectin, laminin, entactin, fibrin, and perlecan.

15. The method of any of the preceding claims, wherein

i) the extracellular matrix-binding activity is non-specific extracellular matrix-binding activity, and/or
ii) the antibody is an IgG antibody,
and/or
iii) wherein measurement of the extracellular matrix-binding activity is measurement by an electrochemiluminescence method.

FIG. 1

FIG. 2

FIG. 3

FIG. 4

FIG. 5

FIG. 6

FIG. 7

FIG. 8

FIG. 9

FIG. 10

Europäisches
Patentamt

European
Patent Office

Office européen
des brevets

# EUROPEAN SEARCH REPORT

Application Number

EP 19 15 5042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | BELDER DE M A ET AL: "ABCIXIMAB (REOPRO TM): A CLINICALLY EFFECTIVE GLYCOPROTEIN IIB/IIIARECEPTOR BLOCKER", EXPERT OPINION ON INVESTIGATIONAL DRUGS, INFORMA HEALTHCARE, UK, vol. 7, no. 10, 1 October 1998 (1998-10-01), pages 1701-1717, XP000934141, ISSN: 1354-3784, DOI: 10.1517/13543784.7.10.1701 * page 1707-1708, figure 1 * | 1-3,8,9 | INV. C07K16/36 C12P21/02 C12P21/08 C12Q1/02 G01N33/53 A61K39/395 A61P37/06 |
| X | C. ANDREW BOSWELL ET AL: "Effects of Charge on Antibody Tissue Distribution and Pharmacokinetics", BIOCONJUGATE CHEMISTRY, vol. 21, no. 12, 15 December 2010 (2010-12-15), pages 2153-2163, XP055046782, ISSN: 1043-1802, DOI: 10.1021/bc100261d * abstract pages 2156-2159 * | 1-15 | |
| A | WEINREB P H ET AL: "A Cell-Free Electrochemiluminescence Assay for Measuring beta1-Integrin-Ligand Interactions", ANALYTICAL BIOCHEMISTRY, ACADEMIC PRESS INC, NEW YORK, vol. 306, no. 2, 15 July 2002 (2002-07-15), pages 305-313, XP027227055, ISSN: 0003-2697 [retrieved on 2002-07-15] * abstract figures * | 15 | TECHNICAL FIELDS SEARCHED (IPC) C07K |

-/--

The present search report has been drawn up for all claims

| Place of search | Date of completion of the search | Examiner |
|---|---|---|
| Munich | 31 July 2019 | Bernhardt, Wiebke |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

Europäisches
Patentamt

European
Patent Office

Office européen
des brevets

# EUROPEAN SEARCH REPORT

Application Number

EP 19 15 5042

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | WO 00/75319 A1 (REGENERON PHARMA [US]; PAPADOPOULOS NICHOLAS J [US]; DAVIS SAMUEL [US]) 14 December 2000 (2000-12-14) * examples, figures * ----- | 1-15 | |
| A | US 2003/129703 A1 (ECONOMIDES ARIS [US] ET AL) 10 July 2003 (2003-07-10) * example 9 paragraph [0256] * ----- | 1-15 | |

TECHNICAL FIELDS
SEARCHED      (IPC)

The present search report has been drawn up for all claims

| Place of search | Date of completion of the search | Examiner |
|---|---|---|
| Munich | 31 July 2019 | Bernhardt, Wiebke |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 19 15 5042

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2019

| Patent document cited in search report | | Publication date | Patent family member(s) | | Publication date |
|---|---|---|---|---|---|
| WO 0075319 | A1 | 14-12-2000 | AT | 293164 T | 15-04-2005 |
| | | | AT | 417928 T | 15-01-2009 |
| | | | AU | 779303 B2 | 13-01-2005 |
| | | | AU | 2005201365 A1 | 28-04-2005 |
| | | | BE | 2013C029 I2 | 06-03-2019 |
| | | | BR | 0011407 A | 02-04-2002 |
| | | | BR | PI0011407 B1 | 25-09-2018 |
| | | | CA | 2376379 A1 | 14-12-2000 |
| | | | CN | 1369009 A | 11-09-2002 |
| | | | CN | 101433715 A | 20-05-2009 |
| | | | CN | 103349781 A | 16-10-2013 |
| | | | CY | 1108883 T1 | 02-07-2014 |
| | | | CZ | 303656 B6 | 30-01-2013 |
| | | | CZ | 20014387 A3 | 16-10-2002 |
| | | | DE | 60019415 T2 | 09-03-2006 |
| | | | DK | 1183353 T3 | 08-08-2005 |
| | | | DK | 1544299 T3 | 23-03-2009 |
| | | | EP | 1183353 A1 | 06-03-2002 |
| | | | EP | 1544299 A1 | 22-06-2005 |
| | | | ES | 2237429 T3 | 01-08-2005 |
| | | | ES | 2319305 T3 | 06-05-2009 |
| | | | HK | 1043388 A1 | 10-06-2005 |
| | | | HK | 1132653 A1 | 13-09-2013 |
| | | | HK | 1185798 A1 | 27-11-2015 |
| | | | HR | P20010908 A2 | 30-04-2005 |
| | | | HU | 230159 B1 | 28-09-2015 |
| | | | HU | 0201515 A2 | 28-08-2002 |
| | | | HU | S1300052 I1 | 29-08-2016 |
| | | | IL | 146890 A | 08-07-2008 |
| | | | JP | 4723140 B2 | 13-07-2011 |
| | | | JP | 5273746 B2 | 28-08-2013 |
| | | | JP | 2003501089 A | 14-01-2003 |
| | | | JP | 2011024595 A | 10-02-2011 |
| | | | LU | 92195 I2 | 15-07-2013 |
| | | | ME | P3208 A | 10-02-2010 |
| | | | ME | 00024 B | 10-02-2010 |
| | | | MX | PA01012630 A | 22-07-2002 |
| | | | NO | 330775 B1 | 11-07-2011 |
| | | | NO | 332559 B1 | 29-10-2012 |
| | | | NO | 2013010 I1 | 17-06-2013 |
| | | | NZ | 515913 A | 30-01-2004 |
| | | | PL | 352246 A1 | 11-08-2003 |
| | | | PT | 1183353 E | 30-06-2005 |
| | | | PT | 1544299 E | 18-03-2009 |
| | | | SK | 17522001 A3 | 01-04-2003 |
| | | | UA | 74146 C2 | 15-11-2005 |

EPO FORM P0459

For more details about this annex : see Official Journal of the European Patent Office, No. 12/82

page 1 of 2

**ANNEX TO THE EUROPEAN SEARCH REPORT
ON EUROPEAN PATENT APPLICATION NO.**

EP 19 15 5042

This annex lists the patent family members relating to the patent documents cited in the above-mentioned European search report.
The members are as contained in the European Patent Office EDP file on
The European Patent Office is in no way liable for these particulars which are merely given for the purpose of information.

31-07-2019

| Patent document cited in search report | Publication date | Patent family member(s) | Publication date |
|---|---|---|---|
| | | WO 0075319 A1 | 14-12-2000 |
| | | YU 86901 A | 12-05-2004 |
| | | ZA 200110068 B | 06-12-2002 |
| US 2003129703 A1 | 10-07-2003 | AT 252639 T | 15-11-2003 |
| | | AU 735851 B2 | 19-07-2001 |
| | | CA 2296769 A1 | 28-01-1999 |
| | | DE 69819167 T2 | 22-07-2004 |
| | | EP 1003860 A1 | 31-05-2000 |
| | | HK 1026229 A1 | 13-02-2004 |
| | | IL 134042 A | 10-12-2006 |
| | | JP 4181300 B2 | 12-11-2008 |
| | | JP 2001510044 A | 31-07-2001 |
| | | US 6075007 A | 13-06-2000 |
| | | US 6500640 B1 | 31-12-2002 |
| | | US 2003129703 A1 | 10-07-2003 |
| | | WO 9903996 A1 | 28-01-1999 |

EPO FORM P0459

For more details about this annex : see Official Journal of the European Patent Office, No. 12/82

page 2 of 2

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2009128931 A
- EP 125023 A
- WO 9602576 A
- WO 2004058821 A
- WO 2003002609 A
- WO 2005037989 A
- WO 2002020565 A
- WO 1995001937 A
- WO 2004044011 A
- WO 2005040229 A
- WO 2002032925 A
- WO 2005035756 A
- WO 2006109592 A

**Non-patent literature cited in the description**

- *Proc. Natl. Acad. Sci.,* 2002, vol. 99 (17), 11393-11398
- **KABAT et al.** Sequence of Proteins of Immunological Interest. National Institute of Health, 1987
- **CHOTHIA et al.** *Nature,* 1989, vol. 342, 877
- *Nat Biotechnol.,* September 2005, vol. 23 (9), 1126-36
- *Current Opinion in Biotechnology,* 2006, vol. 17, 653-658
- *Current Opinion in Biotechnology,* 2007, vol. 18, 1-10
- *Current Opinion in Structural Biology,* 1997, vol. 7, 463-469
- *Protein Science,* 2006, vol. 15, 14-27
- **BAKER MP.** *Carr FJ.Curr Drug Saf.,* 2010, vol. 5 (4), 308-13
- **GUIOTTO et al.** *Bioorg. Med. Chem.,* 2004, vol. 12, 5031-5037
- **KUNKEL.** *Proc. Natl. Acad. Sci. USA,* 1985, vol. 82, 488
- *Mol Cell Biol.,* 1988, vol. 8, 466-472
- Current protocols in Molecular Biology. Publish. John Wiley & Sons, 1987
- Mol Cell Biol. 1988, vol. 8, 466-472
- **VICKI GLASER.** *SPECTRUM Biotechnology Applications,* 1993
- **EBERT et al.** *Bio/Technology,* 1994, vol. 12, 699-702
- **MA et al.** *Eur. J. Immunol.,* 1994, vol. 24, 131-138
- **COX KM et al.** *Nat. Biotechnol.,* December 2006, vol. 24 (12), 1591-1597
- Strategies for Protein Purification and Characterization: A Laboratory Course Manual. Cold Spring Harbor Laboratory Press, 1996
- **GUIOTTO, A. et al.** *Bioorg. Med. Chem.,* 2004, vol. 12, 5031-5037